# EUROPÄISCHE PATENTSCHRIFT

(11) **EP 0 298 268 B1**
(45) Veröffentlichungstag und Bekanntmachung des Hinweises auf die Patenterteilung: **17.05.1995**
(21) Anmeldenummer: 88109226.6
(22) Anmeldetag: 09.06.1988
(51) Int. Cl.: A61N 1/20

(54) **Punktier- und/oder Katheterisiervorrichtung mit einer Punktiernadel für Nervensuchvorgänge**
Device for puncturing and/or catheterizing with a puncture needle for nerve search operations
Dispositif de ponction et/ou cathétérisme avec une aiguille de ponction pour des opérations de recherche de nerfs

(30) Priorität: 10.06.1987 DE 3719353
(43) Veröffentlichungstag der Anmeldung: 11.01.1989
(73) Patentinhaber: STERIMED GESELLSCHAFT MBH, D-66121 Saarbrücken (DE)
(72) Erfinder: Maerz, Dr. Peter, D-8122 Penzberg (DE); Kilian, Gerd, D-6600 Saarbruecken 3 (DE)
(74) Vertreter: Hansmann, Axel, Dipl.-Wirtsch.-Ing.

(56) Entgegenhaltungen:
- EP-A- 0 173 419
- EP-A- 0 211 822
- EP-A- 0 217 011
- DE-A- 1 489 686
- DE-A- 3 006 797
- DE-B- 2 448 444
- GB-A- 2 123 698
- US-A- 4 392 496
- Prospekt der Hugo Sachs Elektronik, March-Hugstetten "Neurostim LA II"

## Beschreibung

Die Erfindung betrifft eine Punktier- und/oder Katheterisiervorrichtung nach dem Oberbegriff des Anspruchs 1.

Derartige Punktier- und/oder Katheterisiervorrichtungen sind bereits bekannt.

Mit solchen Vorrichtungen wird die Punktiernadel für Anästhesiezwecke, Nervenblockaden etc. durch den behandelnden Arzt an einen Nerven herangeführt und es werden dabei die Muskel-Kontraktionen beobachtet, welche durch die Stimulationsimpulse hervorgerufen werden. Daraus werden Schlüsse über die Lage der Punktiernadel oder einer Kanüle zum Nerven gezogen. Elektrische Stimulatoren für Nerven gibt es seit mehr als 70 Jahren (1912). (Münchener Medizinische Wochenschrift Jahrgang 1912, Nr. 47, Seite 2545).

Die am Markt befindlichen Geräte sind verstellbar von 0 bis 10 mA. Es besteht durchwegs das Bestreben und die Voraussetzung, daß die Stimulationsimpulse bei einem Vorschubvorgang der Punktiernadel konstant bleiben.

Unter dieser Voraussetzung kann der anwendende Arzt zunächst bei höherer und dann immer niedrigerer konstanter Sollwerteinstellung der Stromstärke mit der Punktiernadel immer dichter an den Nerv herangehen und aus den dabei jeweils stärker werdenden Muskelkontraktionen die Nähe zum Nerven abschätzen. Das Vorschieben der Punktiernadel erfolgt dabei in Längsrichtung des Nerven durch die Wandung der Nervenscheide und soll beendet werden, wenn die Spitze der Nadel z.B. den Zwischenraum zwischen Nervenscheide und Nerven erreicht hat. Alsdann kann Anästhesiermittel od. dgl. eingespritzt werden. Nach dem Einstechen der Punktiernadel wird am Beginn des Vorschiebens zunächst ein vergleichsweise hoher Strom mit einem angestrebten Sollwert von beispielsweise 10 mA eingestellt und die Einstellung konstant gehalten. Wenn bei weiterem Vorschieben die Muskelkontraktionen des Patienten stärker werden, geht der Arzt mit dem eingestellten Sollwert der Stromstärke zurück und schiebt die Nadel bei gleich gehaltener Sollstrom-Einstellung wieder weiter vor.

Für eine bestimmte Einstechtiefe der Nadel ergibt sich bei einer Verminderung des eingestellten Sollwertes der Stromstärke jeweils auch eine Verminderung der Muskelkontraktionen, die dann bei erneutem weiteren Einstechen der Punktiernadel wieder heftiger werden. Auf diese Weise kann der behandelnde Arzt im Wechsel zwischen Vorschieben der Punktiernadel bei gleichgehaltener Sollwerteinstellung der Stromstärke und Beobachtung der Muskelreaktion einerseits und nachfolgender Herabsetzung des dann wieder gleichgehaltenen Sollwertes der Stromstärke langsam bis dicht an den Nerven herangehen.

Solange der Arzt bei relativ hoher Sollwert-Einstellung eine nur geringfügige Muskelkontraktion beim Patienten feststellt, geht er davon aus, daß die Nadel weiter vorgeschoben werden kann, bis sich stärkere Muskelkontraktionen zeigen. Er stellt dann den Sollwert der Stromstärke weiter zurück und wiederholt den Vorgang.

Bei diesem Vorgehen haben sich nun aber doch immer wieder Nervenverletzungen ergeben, indem der behandelnde Arzt bei relativ hoher eingestellter Stromstärke nur geringfügige Musikelkontraktion feststellte, die Nadel dann weiter vorschob und den Nerven wider Erwarten schon verletzte.

Der vorliegendenErfindung liegt die Aufgabe zugrunde, bei einem Gerät der eingangs als bekannt vorausgesetzten Art Vorkehrungen zu treffen, um unerwünschte Nervenverletzungen mit größerer Sicherheit als bisher ausschließen zu können.

Zur Lösung dieser Aufgabe wird ausgehend von einer Punktier- und/oder Katheterisiervorrichtung der eingangs als bekannt vorausgesetzten Art erfindungsgemäß die Anordnung gemäß Kennzeichen des Anspruches 1 getroffen.

Die dem Stande der Technik angehörenden Geräte waren in der Weise aufgebaut, daß bei der Eichung der Stromstärkenanzeigen zur Einstellung der gewünschten Sollwerte werksseitig ein Testwiderstand auf der Grundlage eines mittleren Widerstandes in der Körpergewebe-Strecke zwischen Haut-Klebelektrode und Punktiernadel-Elektrode zugrunde gelegt wurde. Immer wieder haben sich dabei unerwarteter Weise die oben beschriebenen Verletzungen ergeben, bei denen der Arzt aufgrund nur geringfügiger Muskelkontraktionen bei vergleichsweise noch hoher Sollwert-Einstellung der Stomstärke einen noch größeren Abstand vom Nerven vermutete und den Nerven beim Vorschieben der Nadel dann doch verletzte.

Mit der erfindungsgemäßen Ausgestaltung wird sichergestellt, daß der behandelnde Arzt zusätzlich zu der Sollstrom-Einstellung und ihrer ständigen Ablesung die Möglichkeit zur ständigen Überprüfung der Istrom-Einstellung hat. Wider Erwarten hat sich gezeigt, daß der tatsächliche Iststrom häufig wesentlich niedriger ist als der eingestellte Sollstrom. Ist derartiges der Fall, dann ist nur geringfügige Muskelkontraktion entgegen der bisherigen Annahme kein Zeichen für noch vergleichsweise weiten Abstand vom Nerv, sondern es befindet sich vielmehr die Nadel schcn sehr dicht am Nerv und der Stimulationsstrom bewirkt dennoch nur eine geringfügige Muskelkontraktion.Die bisher immer wieder unvermeidbare fälschliche Auslegung dieser geringen Muskelkontraktion als weiter Abstand vom Nerv wird mit der vorliegenden Erfindung nun zuverlässig vermieden und sichergestellt, daß ein unerwünschtes Einstechen in den Nerv nicht mehr passiert.

Die Unteransprüche kennzeichnen vorteilhafte Weiterbildungen und Ausgestaltungen der Erfindung.

Bei der vorliegenden Erfindung ist es auch das Vorhandensein sowohl einer Anzeige des eingestellten Stromsollwertes als auch einer Messung des tatsächlich fließenden Iststromes, welches es dem behandelnden Arzt jeweils ermöglicht, während der Behandlungsdauer aufgetretene Änderungen, beispielsweise das Austrocknen der Haut-Klebeelektrode, sofort zu realisieren und für Abhilfe zu sorgen.

Steht dem behandelnden Arzt hingegen nur die Sollwert-Einstellung oder nur der Iststrom zur Verfügung, so erkennt der behandelnde Arzt eine eingetretene Änderung der elektrischen Verhältnisse nicht und ist in Gefahr, aus der jeweiligen Sollwertanzeige oder Iststromanzeige allein falsche Rückschlüsse zu ziehen.

Zum Stand der Technik sei noch bemerkt, daß Stromstärkenvorwähler und Iststrom-Messungen bei verschiedenen medizinischen Behandlungsgeräten, insbesondere bei Akupunkturgeräten bereits bekannt geworden sind. Es wird verwiesen insbesondere auf die DE-PS 24 62 934, DE-GM 76 22 099, DE-OS 25 38 289, DE-OS 14 89 686, WO 84/01516, DE-PS 28 43 724, DE-AS 2 443 913.

Keines dieser bekannten Geräte hat jedoch Anhaltspunkte dafür gegeben, wie die der vorliegenden Erfindung zugrunde liegende Aufgabenstellung für eine Punktier und Katheterisiervorrichtung der oben angegebenen Gattung zu lösen sei.

Zum Stande der Technik sind weiterhin auch zu erwähnen die
**EP-A-211 822**
**De-A-2 448 444**
**DE-A-3 3 006 797**
**De-A-1 489 686**
**EP-A-217 011.**

Von diesen Dokumenten betrifft die EP-A 211 822 ein elektromedizinisches Therapiegerät zur nichtmedikamentösen Behandlung von Herpes-Erkrankungen durch Inaktivierung von Herpesviren in organischem Gewebe. Während bei diesem Gerät bereits eine Einheit zur Konstantstromerzeugung und Messung und Anzeige der Stärke des mittels Tastensteuerung zu vergrößernden bzw. zu verkleinernden Behandlungsstromes vorgesehen sind, sind die Erfordernisse einer Herpesbehandlung ganz andere als bei einem Stimulator-Stromkreis für mit einer Punktiernadel-Elektrode durchzuführende Nervensuchvorgänge. Sowohl von der Aufgabenstellung her als auch von den konstruktiven Lösungsmerkmalen her, konnte dieses Dokument daher dem Fachmann keine Anregungen zur Lösung der der Erfindung zugrundeliegenden Aufgabe geben und auch keinen Anlaß, Anregungen an dieser Stelle zu suchen.

Auch die weiteren, vorstehend genannten Druckschriften haben die vorliegende Erfindung weder vorwegnehmen noch nahelegen können. Sie betreffen in anderen Zusammenhängen technische Einzelheiten, für die selbständiger Schutz in der vorliegenden Anmeldung nicht beansprucht wird. So offenbart die DE-A 3 006 797 bei einem Reizstromgerät einen Wählschalter, um die Stärke eines Impulses auf dem einen oder anderen Kanal je nach gewünschter Reizwirkung einstellen zu können (Seite 10, Absatz 4 und 5).

Die DE-A- 1 489 686 offenbart bei einem elektromedizinischen Reizstromgerät für die Zwecke der Schlaftherapie ein Strommeßgerät, mit dessen Hilfe man den im Elektrodenstromkreis fließenden Strom überwachen und die richtige Einstellung eines Potentiometers finden kann, wobei das Meßinstrument sich über einen zweipoligen Umschalter über einen Vorwiderstand an die Batterie legen läßt, so daß auch die Spannung der Batterie mit dem Meßgerät überwacht werden kann.

Die EP-A- 217 011 offenbart bei einem universellen Elektrotherapiegerät zur Erzeugung unterschiedlicher Reizströme, Interferenzströme oder Magnetfelder bereits einen Impulsgenerator, der über Tasten einen Zähler zum Vor- oder Rückwärtszählen veranlaßt, so daß mit den Tasten beliebige Stromstärken eingestellt werden können sollen.

Es folgt die Beschreibung von Ausführungsbeispielen der Erfindung anhand einer Zeichnung.
- Fig. 1: zeigt eine Punktier- und Kathetisiervorrichtung für Nervensuchvorgänge mit schematisch angedeutetem Armbereich des Patienten und einem Blockschaltbild eines elektrischen Stimulator-Stromkreises
- Fig. 2: zeigt eine abgewandelte Ausführungsform mit kombinierter Anzeige für Sollstrom und Iststrom.

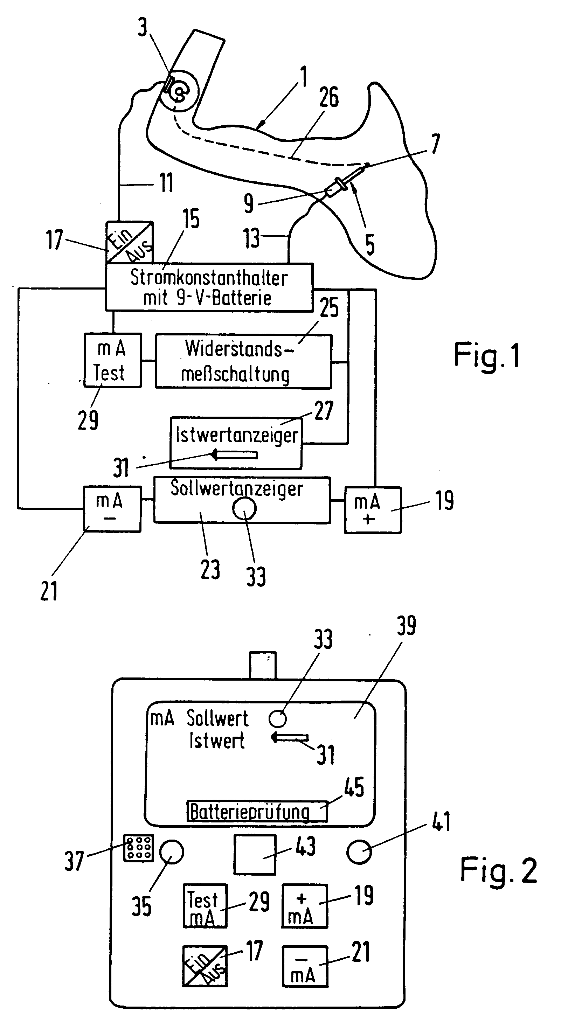

Fig. 1 zeige eine Punktier- und Kathetisiervorrichtung für Nervensuchvorgänge mit schematisch angedeutetem Armbereich des Patienten und einem Blockschaltbild eines elektrischen Stimulator-Stromkreises. Wie dargestellt hat der Patient am Unterarm eine handelsübliche Haut-Klebeelektrode 3 angelegt, wie sie aus der EGK-Meßtechnik bekannt ist, und im Achselbereich des Patienten ist eine Punktiernadel-Elektrode 5 gezeigt, die eine abisolierte Spitze 7 hat und auch für Anästhesiezwecke u. dgl. handelsüblich ist.

Der behandelnde Arzt sticht eine solche Punktiernadel im Achselbereich des Patienten in die Nachbarschaft eines zu suchenden Nerven ein, wobei er sich mit der Punktiernadel allmählich fortschreitend an den Nerv herantastet, ohne diesen selbst zu verletzen. Die Punktiernadel kann am rückwärtigen Ende mit einem Luer®-Konus 9 zum Anschluß einer Spritze für Anästhesiermittel od. dgl. ausgestattet sein. Die Haut-Klebeelektrode 3 und die Punktiernadel 7 sind über elektrische Leitungen 11 und 13 an einen Strom-Konstanthalter 15 mit passender Batterie, beispielsweise 9 Volt angeschlossen. Dieser gestattet es, bei Änderungen der elektrischen Verhältnisse wie sinkender Batteriespannung, veränderten Widerständen, eine Nachführung eines eingestellten Stromes vorzunehmen, so lange die Leistung der Batterie reicht.

Die Sicherheitsvorschriften begrenzen die zulässige Höchstspannung auf 24 Volt, und das Gerät hat einen Soll-Einstellbereich des abgegebenen Stromes zwischen 0,1 und 10 mA.

In nicht näher dargestellter Weise ist das Gerät für die periodische Abgabe negativer Stromimpulse der oben angegebenen Stromstärken ausgelegt. Die Dauer der negativen Impulse beträgt etwa eine Millisekunde, und die Frequenz der Impulse 1 Impuls/Sekunde. Das Gerät ist auch dafür ausgelegt, in der Zeit zwischen zwei Impulsen, die Energie die während des Impulses angelegen hatte, mit entgegengesetzter Polung abzugeben, um so unerwünschte Aufladungen zu vermeiden.

Das Gerät kann mittels eines Ein/Aus-Drucktastenschalters 17 betätigt werden, und es ist weiterhin mit einem Drucktastenschalter 19 für positive Stromstärkenänderung und einem Druckschalter 21 für negative Stromstärkenänderung ausgestattet. Dabei ist auch ein Stromstärken-Sollwertanzeiger 23 vorgesehen. Die Drucktastenschalter 19, 21 für die Stromstärkenänderung sind in nicht näher dargestellter Weise mit Auto-Repeater ausgestattet, so daß bei Niederdrücken des betreffenden Schalters je Sekunde fünf Schaltstufen von je 0,1 mA durchlaufen werden, bis der Schalter wieder losgelassen wird.

Die Drucktastenschalter bilden also einen Sollstromstärken-Vorwähler. Der beschriebene Stimulatorkreis ist dabei für die Sollstromstärken-Anzeige werksseitig mittels eines Testwiderstandes eingestellt, der einem Druchschnittswert der bei Nerven-Stimulier-Vorgängen angetroffenen Widerstände entspricht.

Ein Gerät dieser Art gestattet es dem behandelnden Arzt nach Anlegen bzw. Einstechen der Elektroden 3 und 5 die durch die abgegebenen Stromimpulse erzeugten Muskelkontraktionen des Patienten zu beobachten und daraus Rückschlüsse auf die Lage der Spitze der Punktiernadel zum Nerv zu ziehen.

Zusätzlich zu der beschriebenen Anzeigevorrichtung für die gewünschten Sollstromstärken, ist der Stimulatorstromkreis nun mit einer Einrichtung 25 für eine Iststrom-Messung in der Körpergewebe-Strecke 26 zwischen Haut-Klebeelektrode 3 und Punktiernadel-Elektrode 5 ausgestattet. Bei dieser Vorrichtung kann es sich um eine in der Elektrotechnik bekannte Widerstandsmeßschaltung handeln. Sie ist auch mit einem Anzeiger 27 für die gemessene Iststromstärke ausgestattet. Dabei ist die Anordnung vorzugsweise derart getroffen, daß der Anzeiger 27 für den Iststrom den tatsächlichen Iststrom in der Körpergewebestrecke 27 ohne den von den Bestandteilen der Meßschaltung aufgenommenen Strom anzeigt. Der Stimulatorstromkreis ist also für Iststrom-Messungen in der Körpergewebe-Strecke zwischen Haut-Klebeelektrode und Punktiernadel-Elektrode ausgelegt.

Zum Einschalten der Einrichtung 25 für die Iststrom-Messung dient ein Drucktastenschalter 29, der als Testschalter bezeichnet werden kann. Mittels dieses als Testschalter dienenden Drucktastenschalters 29 wird zugleich ein im Bereich des Anzeigers 27 für den Iststrom vorgesehenes optisches Signalzeichen in Form eines Leuchtpfeiles 31 geschaltet. Auf diese Weise kann der behandelnde Arzt auf einen Blick erkennen, ob eine Sollstrom-Anzeige oder die gewünschte Iststrom-Anzeige gegeben wird.

Im Zusammenhang mit dem Anzeiger 23 für den Sollstrom kann im übrigen noch eine rote Warnleuchte 33 angeordnet sein, welche aus sicherheitstechnischen Gründen anspricht, wenn die Iststromstärke die gewünschte Sollstromstärke überschreitet, und aus den gleichen Gründen kann die Warnleuchte mit einem selbsttätigen Ausschalter gekuppelt sein.

Eine weitere grüne Anzeigeleuchte 35 kann im Takt der abgegebenen Stromimpulse geschaltet und auch mit einem akustischen Signalgeber 37 gekoppelt sein.

Bei der in Fig. 2 gezeigten Ausführungsform der Erfindung sind die Anzeiger 23 für den Sollstrom und 27 für den Iststrom in einem einzigen digitalen Anzeigegerät 39 zusammengefaßt, welches auch das als Pfeil 31 ausgeführte optische Signalzeichen enthält. Drückt die Bedienungsperson die als Testtaste dienende Drucktaste 29, so erscheint die Anzeige des Iststromes zusammen mit einem Aufleuchten des Pfeiles 31. Die Anzeige des Sollstromes ist mit dem Ein/Aus-Drucktastenschalter und den Drucktastenschaltern 19 und 21 für die Stromstärkenveränderung gekoppelt. Die Verzögerungszeit zwischen einer Veränderung des Sollstromes und der Anzeige in der Anzeigevorrichtung läßt sich auf 0,1 Sekunden begrenzen. Es ist auch ein automatischer Ausschalter vorgesehen, der das Gerät selbständig abschaltet, wenn 15 Minuten lang kein Strom fließt. Der Stromverbrauch des Gerätes läßt sich auf 5 mA oder geringer begrenzen, so daß sich bei einer Alkali-Batterie ca. 100 Betriebsstunden ergeben.

Eine Batterieprüftaste 43 schaltet ein Batterieprüfgerät und eine Anzeige 45 "Batterieprüfung" im Anzeigegerät 39.

Das beschriebene Gerät stellt zuverlässig sicher, daß der behandelnde Arzt nicht falschen Annahmen über die tatsächliche Lage der Punktiernadelspitze unterliegen kann. Es wird somit sichergestellt, daß ein unerwünschtes Anstechen eines Nerven vermieden wird. Eine weitere Warnleuchte 41 leuchtet zweckmäßigerweise auf, wenn der Iststrom den Sollstrom unterschreitet und Gefahr unbeabsichtigter Nervenverletzung besteht.

Der Stimulator kann auch zusätzlich eine Betriebsspannungsprüftaste aufweisen, die bei Unterschreiten einer bestimmten Betriebsspannung eine Signallampe zum Aufleuchten bringt. Zweckmäßigerweise leuchtet die Signallampe automatisch dann auf, wenn die Stimulator-Ausgangsspannung unter etwa 10 Volt, vorzugsweise unter 13 Volt absinkt. Diese Spannungswerte sind nicht von der Funktionstüchtigkeit des Stimulators bzw. dessen Schaltungsanordnung abhängig, sondern von den Bedingungen, unter denen der Stimulator effektiv eingesetzt werden kann.

Hinsichtlich der Ausgestaltung des Stimulator-Stromkreises können im einzelnen folgende Vorkehrungen getroffen sein:

Die von der Batterie abgegebene Spannung wird von 9 auf 18 Volt verdoppelt. Für die Schaltung geht etwa 1 Volt verloren, so daß eine Versorgungsspannung von 17 Volt übrig bleibt.

Diese Versorgungsspannung liegt an einem Operationsverstärker an, der die Stromstärke am Ausgang (über einen 100 Ohm Meßwiderstand) mißt, mit dem eingestellten Wert (Soll-Strom) vergleicht und die Versorgungsspannung soweit erhöht oder erniedrigt, bis genau der eingestellte Wert erreicht ist. D.h., wenn der Außenwiderstand (Patient) größer wird, stellt der Operationsverstärker mehr Versorgungsspannung zur Verfügung, wird der Patientenwiderstand kleiner, drosselt der Operationsverstärker die Versorgungsspannung entsprechend dem eingestellten Sollstrom.

Insoweit ist das Gerät als stromkonstant anzusehen, und die Regelung über den Operationsverstärker wäre ein Stromkonstanthalter.

Der Operationsverstärker selbst kann also die Versorgungsspannung in Abhängigkeit vom Lastwiderstand zwischen 0 und 17 Volt regeln, d.h. er kann maximal die gesamte Versorgungsspannung zur Verfügung stellen. Ein weiteres Hochtransformieren ist nicht möglich, da die Versorgungsspannung ja unterhalb der Sicherheitsgrenze bleiben muß.

## Patentansprüche

1. Punktier- und/oder Katheterisiervorrichtung mit einer Punktiernadel, die bis an die Spitze heran außen elektrisch isoliert ist und für Nervensuchvorgänge mit ihrer unisolierten Spitze als Punktiernadelelektrode in Richtung eines Nerven in den Körper einstechbar ist, wobei die Punktiernadel und eine Haut-Klebeelektrode Bestandteile eines auf konstante Impulse einstellbaren Stimulator-Stromkreises sind, der einen Stromstärken-Vorwähler und einen Anzeiger für den eingestellten Sollwert der Stromstärke aufweist, **dadurch gekennzeichnet**, daß der Stimulator-Stromkreis für Iststrom-Messung in der Körpergewebestrecke zwischen Haut-Klebeelektrode und Punktiernadelelektrode ausgelegt ist und mit zusätzlichem Anzeiger (27) auch für den Iststrom (27 bzw. 31) ausgestattet ist.

2. Punktier- und/oder Katheterisiervorrichtung nach Anspruch 1, dadurch gekennzeichnet, daß der zusätzliche Anzeiger (27) für den Iststrom wahlweise an einen mit Widerstandsmeßschaltung (25) ausgestatteten Stromkreiszweig für Iststrom-Anzeige anschließbar ist.

3. Punktier- und/oder Katheterisiervorrichtung nach Anspruch 1 oder 2, dadurch gekennzeichnet, daß die zwischen der Haut-Klebeelektrode (3) und der Punktiernadel-Elektrode (5) liegende Körpergewebe-Strecke (26) und das Strommeßgerät (27) für Iststrom-Messung in Reihe geschaltet sind.

4. Punktier- und/oder Katheterisiervorrichtung nach Anspruch 3, dadurch gekennzeichnet, daß der Einstellbereich einen Istrom-Bereich zwischen 0,1 und 10 mA für Batteriespannungswerte zwischen 5,5 und 9 Volt umfaßt.

5. Punktier- und/oder Katheterisiervorrichtung nach Anspruch 4, dadurch gekennzeichnet, daß ein gemeinsames Anzeigegerät (39) für den eingestellten Sollwert der Stromstärke und für die Anzeige der tatsächlichen Iststromstärke in der Körpergewebe-Strecke auch an ein Batterie-Prüfgerät zur Spannungsprüfung anschließbar ist.

6. Punktier- und/oder Katheterisiervorrichtung nach einem oder mehreren der voranstehenden Ansprüche, dadurch gekennzeichnet, daß das Anzeigegerät ein Digital-Anzeigegerät ist.

7. Punktier- und/oder Katheterisiervorrichtung nach Anspruch 6, dadurch gekennzeichnet, daß das Vorwählgerät zur Einstellung des Sollwertes der Stromstärke mit einem Auto-Repeater mit fünf 0,1 mA-Schaltstufen je Sekunde (Stromstärkensteigerung/Stromstärkensenkung) ausgestattet ist.

8. Punktier- und/oder Katheterisiervorrichtung nach Anspruch 7, dadurch gekennzeichnet, daß das Gerät mit einem Schalter (17) für Ein/Ausschaltung, einem Testschalter (29) zur Einschaltung der Anzeige der Istromstärke, einem Drucktastenschalter (19) für positive Stromstärkenänderung und einem Drucktastenschalter (21) für negative Stromstärkenänderung ausgestattet ist, wobei die Druckschalter für die Stromstärkenänderung zugleich als Einschalter für das Anzeigegerät (23) zur Anzeige des eingestellten Sollwertes der Stromstärke ausgestattet sind.

9. Punktier- und/oder Katheterisiervorrichtung nach einem oder mehreren der voranstehenden Ansprüche, gekennzeichnet durch eine Warnleuchte (33), die auf Überschreiten des eingestellten Sollwertes der Stromstärke durch die gemesssene Iststromstärke anspricht.

10. Punktier- und/oder Katheterisiervorrichtung nach Anspruch 9, gekennzeichnet durch eine weitere Warnleuchte (41), die auf Unterschreiten des eingestellten Sollwertes der Stromstärke durch den Iststrom anspricht.

11. Punktier- und/oder Katheterisiervorrichtung nach einem oder mehreren der voranstehenden Ansprüche, gekennzeichnet durch eine Anzeigenleuchte (35) und einen akustischen Signalgeber (37), die im Takt der abgegebenen Impulse (beispielsweise 1 Impuls/sec.)geschaltet werden.

12. Punktier- und/oder Katheterisiervorrichtung nach einem oder mehreren der voranstehenden Ansprüche, gekennzeichnet durch eine Schaltung zum Einschalten der Anzeige (23, 33) des eingestellten Sollwertes der Stromstärke bei Betätigung des Ein/Aus-Druckschalters (17), Einschalten der Anzeige (27,31) der Iststromstärke bei Betätigung einer Testtaste (29) und Einschalten der Anzeige (23,33) des eingestellten Sollwertes der Stromstärke auch bei Betätigung der Drucktastenschalter (19, 21) für die Stromstärkenänderung.

13. Punktier- und/oder Katheterisiervorrichtung nach einem oder mehreren der voranstehenden Ansprüche, gekennzeichnet durch ein am Anzeigegerät vorgesehenes optisches Signalzeichen (31) welches mit der Anzeige (27) der Iststromstärke gekoppelt ist.

14. Punktier- und/oder Katheterisiervorrichtung nach einem oder mehreren der voranstehenden Ansprüche, gekennzeichnet durch eine selbsttätige Abschaltvorrichtung mit Zeitsteuerung.

15. Punktier- und/oder Katheterisiervorrichtung nach einem oder mehreren der voranstehenden Ansprüche 1 - 14, gekennzeichnet durch eine Betriebsspannungsprüftaste, bei deren Betätigen bei Unterschreiten einer bestimmten Betriebsspannung eine Signallampe aufleuchtet.

16. Punktier- und/oder Katheterisiervorrichtung nach Anspruch 15, dadurch gekennzeichnet, daß die Signallampe bei Absinken der Stimulator-Ausgangsspannung unter 10 Volt, vorzugsweise unter 13 Volt automatisch aufleuchtet.

## Claims

1. Device for puncturing and/or catheterizing having a puncture needle which is externally electrically insulated as far as the tip and is insertable, for nerve search operations, by its uninsulated tip as puncture needle electrode, in the direction of a nerve into the body, the puncture needle and a skin adhesion electrode being components of a stimulator circuit which is settable to constant pulses, which circuit exhibits a current intensity preselector and an indicator for the set theoretical value of the current intensity, characterized in that the stimulator circuit is designed for actual current measurement in the body tissue path between skin adhesion electrode and puncture needle electrode, and is also equipped with an additional indicator (27) for the actual current (27 or 31 respectively).

2. Device for puncturing and/or catheterizing according to Claim 1, characterized in that the selective additional indicator (27) for the actual current is connectable to a circuit branch for actual current indication, which circuit branch is equipped with a resistance measuring circuit (25).

3. Device for puncturing and/or catheterizing according to Claim 1 or 2, characterized in that the body tissue path (26) lying between the skin adhesion electrode (3) and the puncture needle electrode (5) and the current measuring system (27) for actual current measurement are connected in series.

4. Device for puncturing and/or catheterizing according to Claim 3, characterized in that the setting range covers an I current [sic] range between 0.1 and 10 mA for battery voltage values between 5.5 and 9 volts.

5. Device for puncturing and/or catheterizing according to Claim 4, characterized in that a common indicating system (39) for the set theoretical value of the current intensity and for the indication of the real actual-current intensity in the body tissue path is also connectable to a battery test system for voltage testing.

6. Device for puncturing and/or catheterizing according to one or more of the preceding claims, characterized in that the indicating system is a digital indicating system.

7. Device for puncturing and/or catheterizing according to Claim 6, characterized in that the preselector system for the setting of the theoretical value of the current intensity is equipped with an autorepeater having five 0.1 mA switching stages per second (current intensity increase/current intensity reduction).

8. Device for puncturing and/or catheterizing according to Claim 7, characterized in that the system is equipped with a switch (17) for on/off switching, a test switch (29) for energizing the indication of the I current [sic] intensity, a pressure key switch (19) for positive current intensity alteration and a pressure key switch (21) for negative current intensity alteration, the pressure switches for the current intensity alteration being equipped at the same time as energizing switches for the indicating system (23) for the indication of the set theoretical value of the current intensity.

9. Device for puncturing and/or catheterizing according to one or more of the preceding claims, characterized by a warning light (33) which responds to exceeding of the set theoretical value of the current intensity by the measured actual current intensity.

10. Device for puncturing and/or catheterizing according to Claim 9, characterized by a further warning light (41) which responds to falling-below of the set theoretical value of the current intensity by the actual current.

11. Device for puncturing and/or catheterizing according to one or more of the preceding claims, characterized by an indicating light (35) and an acoustic signal generator (37), which are switched in the cycle of the emitted pulses (for example 1 pulse/sec).

12. Device for puncturing and/or catheterizing according to one or more of the preceding claims, characterized by a circuit for energizing the indication (23, 33) of the set theoretical value of the current intensity upon actuation of the on/off pressure switch (17), energization of the indication (27, 31) of the actual current intensity upon actuation of a test key (29) and energization of the indication (23, 33) of the set theoretical value of the current intensity also upon actuation of the pressure key switches (19, 21) for the current intensity alteration.

13. Device for puncturing and/or catheterizing according to one or more of the preceding claims, characterized by an optical signal symbol (31) which is provided on the indicating system and which is coupled to the indication (27) of the actual current intensity.

14. Device for puncturing and/or catheterizing according to one or more of the preceding claims, characterized by an automatic deenergization device with time control.

15. Device for puncturing and/or catheterizing according to one or more of the preceding Claims 1-14, characterized by an operating voltage test key, upon actuation of which a signal lamp illuminates upon falling below a specified operating voltage.

16. Device for puncturing and/or catheterizing according to Claim 15, characterized in that the signal lamp automatically illuminates if the stimulator output voltage falls below 10 volts, preferably below 13 volts.

## Revendications

1. Dispositif pour la réalisation de piqûres et/ou la pose de cathéters, avec aiguille qui est isolée électriquement à l'extérieur jusqu'à sa pointe et qui peut être enfoncée dans le corps en direction d'un nerf, par sa pointe non isolée servant d'électrode d'aiguille, pour des opérations d'étude de nerfs, l'aiguille et une électrode collée sur la peau constituant des composants d'un circuit de courant de stimulation pouvant être réglé sur des impulsions constantes, et qui présente un dispositif de présélection de l'intensité du courant et un affichage de la valeur de consigne réglée de l'intensité du courant, caractérisé en ce que le circuit de courant de stimulation est conçu pour une mesure du courant effectif dans la région des tissus corporels située entre l'électrode collée sur la peau et l'électrode de l'aiguille, et qui est également équipé d'un afficheur supplémentaire (27) pour le courant effectif (27 ou 31).

2. Dispositif pour la réalisation de piqûres et/ou la pose de cathéters selon la revendication 1, caractérisé en ce que l'afficheur supplémentaire (27) pour le courant effectif peut être raccordé à volonté à une branche du circuit de courant équipée d'un circuit (25) de mesure de la résistance, en vue d'affichages du courant effectif.

3. Dispositif pour la réalisation de piqûres et/ou la pose de cathéters selon la revendication 1 ou 2, caractérisé en ce que la région (26) des tissus corporels située entre l'électrode (3) collant sur la peau et l'électrode de l'aiguille (5), ainsi que l'appareil de mesure de courant (27), sont branchés en série pour la mesure du courant effectif.

4. Dispositif pour la réalisation de piqûres et/ou la pose de cathéters selon la revendication 3, caractérisé en ce que la plage de réglage d'une plage de courant effectif s'étend entre 0,1 et 10 mA pour des valeurs de la tension de batterie située entre 5,5 et 9 volts.

5. Dispositif pour la réalisation de piqûres et/ou la pose de cathéters selon la revendication 4, caractérisé en ce qu'un appareil d'affichage commun (39) de la valeur de consigne réglée de l'intensité du courant et de l'intensité instantanée du courant effectif dans la région des tissus corporels peut également être raccordé à un appareil de vérification de la batterie, pour la vérification de la tension.

6. Dispositif pour la réalisation de piqüres et/ou la pose de cathéters selon l'une ou plusieurs des revendications précédentes, caractérisé en ce que l'appareil d'affichage est un appareil d'affichage numérique.

7. Dispositif pour la réalisation de piqûres et/ou la pose de cathéters selon la revendication 6, caractérisé en ce que l'appareil de présélection en vue du réglage de la valeur de consigne de l'intensité du courant est équipé d'un auto-répétiteur à cinq étages de commutation de 0,1 mA par seconde (augmentation de l'intensité du courant/diminution de l'intensité du courant).

8. Dispositif pour la réalisation de piqûres et/ou la pose de cathéters selon la revendication 7, caractérisé en ce que l'appareil est équipé d'un commutateur (17) de branchement et de débranchement, d'un commutateur de test (29) pour le branchement de l'affichage de l'intensité du courant effectif, d'un commutateur à bouton poussoir (19) pour la modification dans le sens positif de l'intensité du courant et d'un commutateur à bouton poussoir (21) pour la modification dans le sens négatif de l'intensité du courant, les commutateurs à bouton poussoir pour la modification de l'intensité du courant étant également équipés comme commutateurs de branchement de l'appareil d'affichage (23) pour l'affichage de la valeur de consigne réglée pour l'intensité du courant.

9. Dispositif pour la réalisation de piqûres et/ou la pose de cathéters selon l'une ou plusieurs des revendications précédentes, caractérisé par un témoin d'avertissement (33) qui répond lorsque la valeur de consigne réglée de l'intensité du courant est dépassée par l'intensité mesurée du courant effectif.

10. Dispositif pour la réalisation de piqûres et/ou la pose de cathéters selon la revendication 9, caractérisé par un autre témoin d'affichage (41) qui répond lorsque la valeur de consigne réglée de l'intensité du courant n'est pas atteinte par le courant effectif.

11. Dispositif pour la réalisation de piqûres et/ou la pose de cathéters selon l'une ou plusieurs des revendications précédentes, caractérisé par un témoin d'affichage (35) et un émetteur de signaux acoustiques (37), qui sont branchés en synchronisme avec les impulsions émises (par exemple 1 impulsion/sec.).

12. Dispositif pour la réalisation de piqûres et/ou la pose de cathéters selon l'une ou plusieurs des revendications précédentes, caractérisé par un circuit de branchement des affichages (23, 33) de la valeur de consigne réglée de l'intensité du courant lorsque l'on actionne le commutateur à touche (17) de branchement et de débranchement, pour le branchement des affichages (27, 31) de l'intensité du courant effectif lorsque l'on actionne une touche de test (29) et pour le branchement des affichages (23, 33) de la valeur de consigne réglée de l'intensité du courant, également lors de l'actionnement des commutateurs à bouton poussoir (19, 21) pour la modification de l'intensité du courant.

13. Dispositif pour la réalisation de piqûres et/ou la pose de cathéters selon l'une ou plusieurs des revendications précédentes, caractérisé par un affichage de signalisation optique (31) prévu sur l'appareil d'affichage, et qui est couplé à l'affichage (27) de l'intensité du courant effectif.

14. Dispositif pour la réalisation de piqûres et/ou la pose de cathéters selon l'une ou plusieurs des revendications précédentes, caractérisé par un dispositif de débranchement automatique à commande temporisée.

15. Dispositif pour la réalisation de piqûres et/ou la pose de cathéters selon une ou plusieurs des revendications précédentes 1-14, caractérisé par une touche de vérification de la tension de fonctionnement, qui lorsqu'elle est actionnée quand une tension de fonctionnement déterminée n'est pas atteinte, allume une lampe de signalisation.

16. Dispositif pour la réalisation de piqûres et/ou la pose de cathéters selon la revendication 15, caractérisé en ce que la lampe de signalisation s'allume automatiquement lorsque la tension de sortie du stimulateur descend en dessous de 10 volts, et de préférence en dessous de 13 volts.
